# FASCICULE DE BREVET EUROPEEN

(11) **EP 1 083 898 B1**
(45) Date de publication et mention de la délivrance du brevet: **04.01.2006**
(21) Numéro de dépôt: 99923725.8
(22) Date de dépôt: 11.06.1999
(51) Int. Cl.: A61K 31/425, A61K 31/47, A61P 31/12

(54) **NOUVELLE UTILISATION DE COMPOSES INHIBITEURS DE LA PROTEASE DU VIH**
NEUE VERWENDUNG VON HIV-PROTEASE HEMMERN
NOVEL USE OF HIV PROTEASE INHIBITING COMPOUNDS

(30) Priorité: 11.06.1998 FR 9807373
(43) Date de publication de la demande: 21.03.2001
(73) Titulaire: INSTITUT NATIONAL DE LA SANTE ET DE LA RECHERCHE MEDICALE (INSERM), 75013 Paris (FR); BIOMERIEUX, 69280 Marcy-L'Etoile (FR)
(72) Inventeur: ANDRE, Patrice, F-35700 Rennes (FR); LOTTEAU, Vincent, F-35000 Rennes (FR); ZINKERNAGEL, Rolf, CH-8126 Zumikon (CH); KLENERMAN, Paul, Oxford OX1 1TQ (GB); GROETTRUP, Marcus, CH-9008 St. Gallen (CH)
(74) Mandataire: Bernasconi, Jean Raymond
(86) Numéro de dépôt international: PCT/FR1999/001391
(87) Numéro de publication internationale: WO 1999/063998

(56) Documents cités:
- EP-A- 0 432 694
- EP-A- 0 432 695
- WO-A-94/14436
- WO-A-96/13266
- WO-A-96/26734
- WO-A-98/52571
- DE-A- 4 307 883
- ANDRE, PATRICE ET AL: "An inhibitor of HIV-1 protease modulates proteasome activity, antigen presentation, and T cell responses" PROC. NATL. ACAD. SCI. U. S. A. (1998), 95(22), 13120-13124 CODEN: PNASA6;ISSN: 0027-8424, XP002095181
- BERTHELOT P ET AL: "Dramatic cutaneous psoriasis improvement in a patient with the human immunodeficiency virus treated with 2',3'-dideoxy,3'-thyacytidine [correction of 2',3'-dideoxycytidine] and ritonavir [letter] [published erratum appears in Arch Dermatol 1998 Apr;134(4):452]." ARCHIVES OF DERMATOLOGY, (1997 APR) 133 (4) 531. JOURNAL CODE: 6WU. ISSN: 0003-987X., XP002095182 United States
- CARR, ANDREW (1) ET AL: "Restoration of immunity to chronic hepatitis B infection in HIV-infected patient on protease inhibitor." LANCET (NORTH AMERICAN EDITION), (1997) VOL. 349, NO. 9057, PP. 995-996. ISSN: 0099-5355., XP002095183
- O.T. RUTSCHMANN ET AL.: "Impact of treatment with Human Immunodeficiency Virus (HIV) Protease Inhibitors on Hepatitis C viremia in Patients Coinfected with HIV" THE JOURNAL OF INFECTIOUS DISEASES, vol. 177, no. 3, mars 1998 (1998-03), pages 783-785, XP002095184
- MAUSS, S. (1) ET AL: "Influence of HIV protease inhibitors on hepatitis C viral load in individuals with HIV and HCV coinfection." ABSTRACTS OF THE INTERSCIENCE CONFERENCE ON ANTIMICROBIAL AGENTS AND CHEMOTHERAPY, (1997) VOL. 37, PP. 218. MEETING INFO.: 37TH INTERSCIENCE CONFERENCE ON ANTIMICROBIAL AGENTS AND CHEMOTHERAPY TORONTO, ONTARIO, CANADA SEPTEMBER 28-OCTOBER 1, 1997 ICA, XP002095185
- AUTRAN, B. ET AL: "Positive effects of combined antiretroviral therapy on CD4+ T cell homeostasis and function in advanced HIV disease" SCIENCE (WASHINGTON, D. C.) (1997), 277(5322), 112-116 CODEN: SCIEAS;ISSN: 0036-8075, XP002095186
- BADLEY, ANDREW D. ET AL: "In vivo analysis of Fas/FasL interactions in HIV-infected patients" J. CLIN. INVEST. (1998), 102(1), 79-87 , XP002121453
- SLOAND, E. M. (1) ET AL: "HIV-1 protease inhibitor modulates activation of peripheral blood derived CD4+T-cells and decreases their susceptibility to apoptosis in vitro and in vivo." BLOOD, (NOV. 15, 1997) VOL. 90, NO. 10 SUPPL. 1 PART 1, PP. 577A. MEETING INFO.: 39TH ANNUAL MEETING OF THE AMERICAN SOCIETY OF HEMATOLOGY SAN DIEGO, CALIFORNIA, USA DECEMBER 5-9, 1997 THE AMERICAN SOCIETY OF HEMATOLOGY. , XP002121454
- KOTLER D.P. ET AL: "Effect of combination antiretroviral therapy upon rectal mucosal HIV RNA burden and mononuclear cell apoptosis." AIDS, (16 APR 1998) 12/6 (597-604). , XP002121455
- DIZ DIOS P. ET AL: "Regression of AIDS-related Kaposi's sarcoma following ritonavir therapy." ORAL ONCOLOGY, (1998) 34/3 (236-238). , XP002121456
- PARRA R. ET AL: "Regression of invasive AIDS-related Kaposi's sarcoma following antiretroviral therapy." CLINICAL INFECTIOUS DISEASES, (1998) 26/1 (218-219). , XP002121457
- M.A. CONANT ET AL.: "REDUCTION OF KAPOSI'S SARCOMA LESIONS FOLLOWING TREATMENT OF AIDS WITH RITONAVIR" AIDS, vol. 11, no. 10, 1997, pages 1300-1301, XP002121458
- REED, J. BRIAN ET AL: "Regression of cytomegalovirus retinitis associated with protease-inhibitor treatment in patients with AIDS" AM. J. OPHTHALMOL. (1997), 124(2), 199-205 , XP002121459

## Description

La présente invention a pour objet l'utilisation thérapeutique d'un composé inhibiteur de la protéase du virus de l'immunodéficience humaine (VIH) choisi parmi le ritonavir, le saquinavir, ou un de leurs sels pharmaceutiquement acceptables, en association avec un véhicule pharmaceutiquement acceptable, pour la fabrication d'un médicament pour la prévention et/ou le traitement d'une infection par le virus de l'hépatite C.

Le protéasome, système enzymatique central de la dégradation protéique à la fois dans le cytosol et dans le noyau, est un complexe présentant de multiples activités peptidasiques. Une de ses fonctions est la génération de petits peptides par protéolyse intracellulaire, ces petits peptides étant présentés aux lymphocytes T pour initier des réponses immunitaires.

Il a été montré que des aldéhydes de peptides sont des inhibiteurs du protéasome et bloquent la dégradation de la plupart des protéines cellulaires et la génération des peptides présentés à la surface des cellules présentatrices d'antigènes, en association avec les molécules du complexe majeur d'histocompatibilité de classe I (Rock et al, Cell, 1994, vol 78, 761-771). Plusieurs demandes de brevet divulguent par ailleurs des inhibiteurs de protéasome utilisables dans le traitement des maladies dans lesquelles une perte de masse corporelle est observée (WO 95/24 914), des maladies résultant dune prolifération cellulaire (WO 98/13 061) et plus généralement des maladies impliquant la fonction protéolytique du protéasome, telles que notamment les maladies inflammatoires et les cancers (WO 96/32 105 ; WO 96/13 266).

De manière surprenante, les auteurs de la présente invention ont découvert que certains composés inhibiteurs de la protéase du virus de l'immunodéficience humaine (VIH) présentent une action modulatrice sur l'activite du protéasome.

Il s agit :
- du ritonavir, et ses sels pharmaceutiquement acceptables, une spécialité pharmaceutique contenant le ritonavir en tant que principe actif, étant le Norvir® (Abbott) ;
- et du saquinavir, et ses sels pharmaceutiquement acceptables, une spécialité pharmaceutique contenant le saquinavir, sous la forme de mésylate de saquinavir en tant que principe actif, étant l'Invirase® (Roche).

Le ritonavir, le saquinavir ou leurs sels peuvent être utilisés seuls ou en mélange. Une association de ritonavir et de saquinavir est particulièrement avantageuse car elle permet d'améliorer la pharmacocinetique du saquinavir, dont la dégradation est ralentie en présence du ritonavir.

Ces inhibiteurs (décris dans les demandes de brevet WO 94/14 436 et EP 432 695) de la protéase du virus de l'immunodéficience humaine (VIH) sont largement utilisés dans le traitement du SIDA. Ces composés bloquent la réplication virale en inhibant spécifiquement la protéase virale qui permet le clivage des précurseurs protéiques viraux en protéines virales matures. La trithérapie mettant en oeuvre un tel inhibiteur de protéase associé à deux analogues de nucléoside est ainsi actuellement la stratégie la plus efficace pour le traitement d'une infection par le VIH.

La présente invention a donc pour objet l'utilisation d'au moins un composé inhibiteur de la protéase du virus de l'immunodéficience humaine (VIH) choisi parmi le ritonavir, le saquinavir, ou un de leurs sels pharmaceutiquement acceptables, en association avec un véhicule pharmaceutiquement acceptable, pour la fabrication d'un médicament pour la prévention et/ou le traitement d'une infection par le virus de l'hépatite C.

Les auteurs de la présente invention ont ainsi découvert que le ritonavir et le saquinavir inhibaient l'activité « chymotrypsin-like » du protéasome et augmentaient l'activité « trypsine » du protéasome.

La modulation du protéasome permet d'intervenir sur un certain nombre d'évenements "en aval".

Les auteurs de la présente invention ont plus particulièrement mis en évidence que ces composés inhibiteurs de la protéase du virus de l'immunodéficience humaine (VIH) présentant une action modulatrice de l'activité du protéasome permettaient de modifier la présentation des antigènes en association avec le complexe majeur d'histocompatibilité de classe I (CMH1), à la surface des cellules et par voie de conséquence inhibaient ou modifiaient l'activation des lymphocytes T cytotoxiques CD8⁺. Ces composés présentent donc l'avantage crucial de ne pas influer directement sur l'activité des lymphocytes T auxiliaires aux concentrations thérapeutiques. Les composés inhibiteurs de la protéase du virus de l'immunodéficience humaine (VIH) présentant une action modulatrice de l'activité du protéasome sont donc particulièrement utiles pour la prévention et/ou le traitement des affections dans lesquelles une réponse inadéquate, par exemple excessive, ces lymphocytes T cytotoxiques CD8⁺ est observée. De manière plus génerale sont visées les affections pour le traitement desquelles une modulation (telle que notamment une diminution) de la réponse immune relayée par les lymphocytes T cytotoxiques CD8⁺ est recherchée.

Les composés inhibiteurs de la protéase du VIH et modulateurs du protéasome selon l'invention présentent par ailleurs une activité de modulation de l'apoptose, conséquence de la modulation du protéasome (Nagata et al, 1997 Cell. 88:355-365 : Ruggieri et al, 1997, Virology, 229:68-76: WO 98/13 061).

Parmi les affections pour lesquelles il est intéressant de moduler l'activité du protéasome, on peut citer notamment les maladies inflammatoires. infectieuses et/ou celles pour le traitement desquelles une modulation ou un contrôle de l'apoptose est désiré. Sont plus particulièrement visés :
- les infections virales notamment les infections par des virus non cytopathogènes telles que les infections par les virus des hépatites, en particulier le virus de l'hépatite C :

Il est entendu que le syndrome de l'immunodeficience acquise (SIDA) n'est pas une affection visée, dans la mesure où la diminution du nombre de lymphocytes T CD8⁺ n'est pas désirée dans le traitement de ce syndrome.

Le médicament préparé conformément à la présente invention et contenant au moins un composé modulateur de l'activité du protéasome, choisi parmi le ritonavir le saqunavir ou de leurs sels pharmaceutiquement acceptable, en association avec un véhicule pharmaceutiquement acceptable, peut être sous la forme d'une composition pharmaceutique destinée à une administration par voie orale, par exemple sous la forme d'un comprimé, d'une géluie, d'une solution buvable, etc. ou par voie rectale, par exemple sous la forme d'un suppositoire. Il peut être également administré par voie parentérale, en particulier sous la forme d'une solution injectable, notamment par voie intraveineuse, intradermique, sous-cutanée, etc. Il peut être enfin sous la forme d'une composition pharmaceutique destinée à une administration topique, telle qu'une pommade. Une telle formulation est particulièrement avantageusement dans le cas du traitement du psoriasis et des hypersensibilités de contact.

Le médicament préparé conformément à la présente invention contient de préférence de 1 à 2000 mg, de préférence de 100 à 500 mg, dudit composé présentant une action modulatrice de l'activité du protéasome.

La présente invention a également trait à une méthode de traitement thérapeutique dans laquelle on administre à un patient souffrant d'une affection pour le traitement de laquelle une modulation du protéasome est désirée, une quantité thérapeutiquement efficace d'au moins un compose inhibiteur de la protéase du VIH choisi parmi le ritonavir, le saquinavir, ou un de leurs sels pharmaceutiquement acceptables, seuls ou en mélange, en association avec un véhicule pharmaceutiquement acceptable.

La posologie dépend de la gravité de l'affection, de l'àge et au poids du patient. Elle peut être notamment de 100 à 1500 mg par jour, de préférence de 600 à 1200 mg par jour.

Rutschmann et al. rapporte l'impact d'un traitement d'un patient coinfecté par le virus VIH et VHC avec des inhibiteurs de la protéase de VIH (ritonavir, indinavir et combinaison de saqinavir et ritonavir). D'après les auteurs, les inhibiteurs de la protéase du VIH n'ont pas d'activité directe sur le VHC et peuvent même aggraver la charge virale du VHC ("Impact of treatment with Human Immunodeficiency Virus (HIV) Protease Inhibitors on Hepatitis C viremia in Patients Coinfected with HIV" THE JOURNAL OF INFECTIOUS DISEASES, vol. 177, no. 3, mars 1998 (1998-03), pages 783-785).

Mauss et al. concerne l'influence d'inhibiteurs de la protéase de VIH sur la charge virale de l'hépatite C chez des individus coinfectés par le VIH et le VHC. Ils constatent qu'au moins le saquinavir et l'indinavir n'ont pas d'effet marqué sur la réplication du VHC ("Influence of HIV protease inhibitors on hepatitis C viral load in individuals with HIV and HCV coinfection." ABSTRACTS OF THE INTERSCIENCE CONFERENCE ON ANTIMICROBIAL AGENTS AND CHEMOTHERAPY, (1997) VOL. 37, PP. 218. MEETING INFO.: 37TH INTERSCIENCE CONFERENCE ON ANTIMICROBIAL AGENTS AND CHEMOTHERAPY TORONTO, ONTARIO, CANADA SEPTEMBER 28-OCTOBER 1, 1997 ICA).

Les figures et les exemples ci-dessous illustrent l'invention.

### LEGENDE DES FIGURES :

- La figure 1A représente l'épaisseur du coussinet plantaire de souris en fonction du temps, les souris traitées par du ritonavir étant comparées a des souris contrôle.
- La figure 1B représente l'épaisseur du coussinet plantaire de souris au 8^{eme} jour après le début du traitement par du ritonavir, les doses quotidiennes de ritonavir étant variables.
- La figure 1C représente le pourcentage de lyse spécifique des cellules cibles EL4 sensibilisées par le peptide GP33-41 par des lymphocytes isolés de rate de souris traitées ou non par du ritonavir.
- La figure 1D représente le pourcentage de lyse spécifique des cellules cibles EL4 sensibilisées par le peptide NP 396-404 par des lymphocytes isolés de rate de souris traitées ou non par du ritonavir.
- La figure 1E représente le pourcentage de cellules CD8⁺ par rapport au nombre total de splénocytes, dans le cas de souris infectées ou non par !e virus LCMV, et traitées ou non par du ritonavir.
- La figure 1 F représente l'effet du ritonavir sur la charge virale en LCMV.
- La figure 2A représente le pourcentage de lyse spécifique des cellules cibles (fibroblastes MC57 infectés par LCMV) incubées en présence ou non de ritonavir, par des lymphocytes T cytotoxiques anti-LCMV.
- La figure 2B représente le pourcentage de lyse spécifique des cellules cibles (fibroblastes MC57 transfectés par la glycoprotéine GP) incubées en présence ou en l'absence de ritonavir, par des lymphocytes T cytotoxiques anti-GP.
- La figure 3A représente le pourcentage maximum de libération de TNFα en fonction de la dose de ritonavir utilisée.
- La figure 3B représente le pourcentage d'inhibition de la prolifération de lymphocytes T M77-84. en fonction de la dose de ritonavir utilisée.
- La figure 4A représente le pourcentage d'inhibition de la dégradation des protéines à courte durée de vie en fonction de la dose de ritonavir utilisée.
- La figure 4B représente l'hydrolyse d'un substrat fluorogène Suc-LLVY-MCA par le protéasome en fonction de la dose de ritonavir utilisée.
- La figure 4C représente l'hydrolyse d'un substrat fluorogène (Z)-GGL-MCA par le protéasome en fonction de la dose de ritonavir utilisée.
- La figure 4D représente l'hydrolyse d'un substrat fluorogène Bz-VGR-MCA par le protéasome en fonction de la dose de ritonavir utilisée.
- La figure 4E représente l'hydrolyse du substrat fluorogène Suc-LLVY-MCA par le protéasome, en présence de nelfinavir, saquinavir, indinavir ou ritonavir,
- La figure 5 représente le pourcentage de souris NOD non diabétiques en fonction du temps, dans le cas d'un traitement par du ritonavir en comparaison avec une administration d'un diluant.
- La figure 6 représente les scores cliniques des rats atteints d'encéphalite allergique expérimentale (EAE), traités par du ritonavir.
- La figure 7 représente l'évolution de l'infection chronique par le virus de l'hépatite C chez un patient infecté par le VIH et traité par du ritonavir.

### EXEMPLES :

### EXEMPLE COMPARATIF 1 :

### Effet du ritonavir sur l'activité des lymphocytes T cytotoxiques in vivo

### 1. Inhibition du gonflement des coussinets plantaires de souris après une infection par le virus LCMV.

### Méthode :

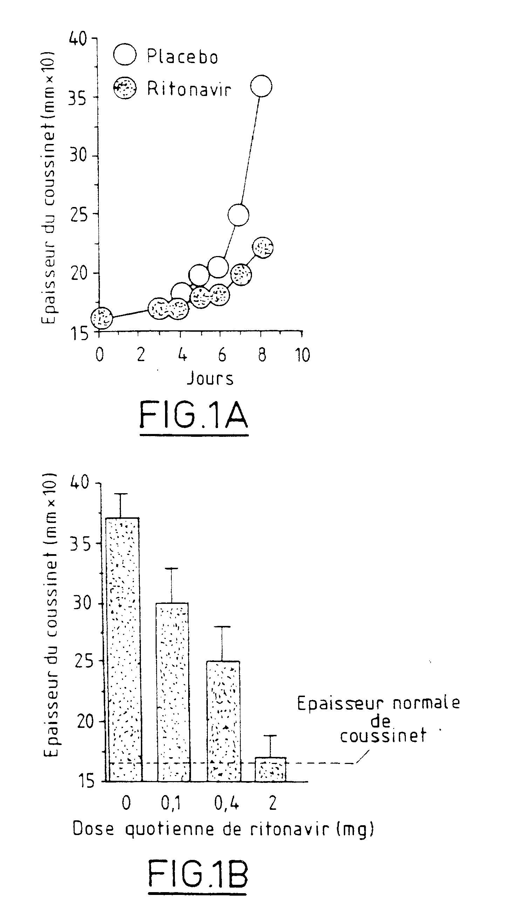

Les auteurs de la présente invention ont utilisé le modèle murin du LCMV (virus de la chorio-méningite lymphocytaire), ce virus non cytopathogène provoquant une infection dans laquelle les lymphocytes T cytotoxiques sont responsables à la fois du contrôle initial de la réplication virale et de l'immunopathologie induite par le virus. Des souris C57BL 6 sont infectées au niveau du coussinet des pattes au jour J0 (300 pfu -unités formatrices de plaques- de LCMV-WE fourni par F Lehmann-Grube, Hambourg) et le gonflement du coussinet de la patte est mesuré au cours du temps. Le ritonavir (1.25 mg/souris/jour dissous dans 10 % d'alcool. 90 % de solution saline de tampon phosphate PBS) ou un placebo (même volume de PBS : 10 % d'alcool) est administre par voie intrapéritonéale à partir du jour J0. Dans cette expérience et dans les expériences suivantes, les résultats sont donnes à partir de deux coussinets des pattes de 2-3 souris par groupe, ces résultats étant représentatifs de trois a quatre expériences séparées. La mesure de l'épaisseur des coussinets de la patte a été réalisée quotidiennement conformément au protocole fourni dans R.M. Zinkernagel. T. Leist, H. Hengartner and A. Althage. J. Exp. Med. 162. 2125 (1985) (figure 1A).

Le gonflement du coussinet induit par l'infection au LCMV a été mesuré au jour J8 chez les souris traitées avec des doses variables de ritonavir (figure 1B).

### Résultats :

Les souris C57BL/6 auxquelles on a injecté le virus LCMV-WE ont présenté, après sept à huit jours, un gonflement du coussinet qui est une mesure directe *in vivo* de l'activité des lymphocytes T cytotoxiques, Ce gonflement a été inhibé de manière marquée par un traitement au ritonavir, d'une manière dose-dépendante. Des résultats similaires ont été obtenus en utilisant différentes souches virales (souche LCMV-Docile 10 pfu/ml obtenue auprès de C. Pfau. Troy ou souche LCMV-Amstrong 10⁴ PFU/ml obtenue auprès de M. Buchmeier La Jolla), avec des souris de différents haplotypes (BALB/c : H2^{d}), et que la voie d'administration soit parentérale ou orale (par sonde intra-gastrique).

### 2. Inhibition de l'activité de lymphocytes T cytotoxiques in vivo. Méthode :

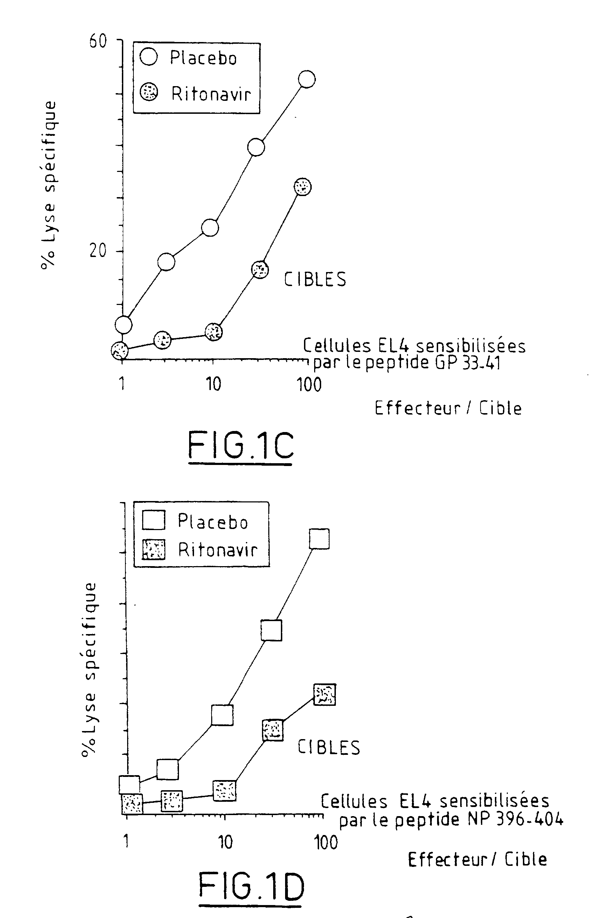

Des souris C57BL/6 traitées par du ritonavir (1.25 mg/souris/jour) ou par un placebo ont été infectées avec 200 PFU de LCMV-WE par voie intraveineuse. Huit jours après l'infection, les splénocytes ont été recueillis et testés pour la lyse des cellules EL4 (H-2^{b}) sensibilisées avec 500 nM du peptide GP33-41 (acides aminés 33 à 41 de la glycoprotéine du LCMV, soit KAVYNFATC : figure 1C) ou du peptide NP 396-407 (acides aminés 396 a 407 de la glycoprotéine du LCMV. soit EQPQNGFIH : figure 1D).

### Résultats :

Le traitement avec du ritonavir a inhibé la réponse des lymphocytes T cytotoxiques a une infection systémique par le virus LCMV. La lyse des cellules cibles a été réduite lorsque les souris ont reçu le traitement par le ritonavir.

### 3 Inhibition de la prolifération des lymphocytes T CD8+ in vivo. Méthode :

Des souris ont été infectées avec 200 PFU de LCMV-WE par voie intraveineuse et traitées avec du ritonavir ou un placebo comme décrit ci-dessus. Les splénocytes ont été identifiés avec un anticorps anti-CD8 conjugué à de la FITC (Pharmingen, San Diego) et analysés par FACS.

### Résultats :

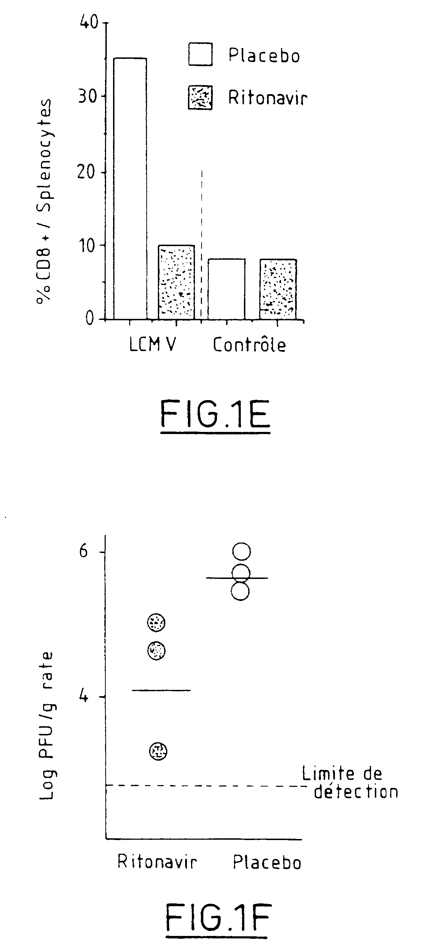

On observe une inhibition de la prolifération normalement observée des lymphocytes T CD8+ en réponse au virus LCMV (figure 1 E).

### 4. Effet du ritonavir sur la charge de LCMV

### Méthode :

Des souris ont été infectées avec un virus LCMV (200 pfu) par voie intraveineuse et traitées avec du ritonavir ou un placebo comme décrit au point 2 ci-dessus. Le titre des virus dans la rate a été déterminé au jour J8, conformément au protocole décrit dans Battegay et al., J. Virol. Methods, 33, 191 (1991).

### Résultats :

La réduction de la lyse des cellules cibles telle qu'observée ci-dessus s'accompagne d'une diminution de la clairance virale (figure 1F), résultat qui confirme que le ritonavir agit directement sur la réponse immunitaire plutôt qu'indirectement par un effet anti-viral sur le LCMV.

### 5. Evaluation de la réponse anticorps.

### Méthode :

Les anticorps spécifiques de la nucléoprotéine du LCMV et de la glycoprotéine du LCMV ont été évalués au bout de quatre et huit semaines par un test ELISA tel que décrit dans O. Planz, P. Seiler, H. Hengartner and R.M. Zinkernagel, Nature. 382. 629 (1990).

### Résultats :

Le ritonavir ne réduit pas la capacité de synthèse d'anticorps anti-virus.

### EXEMPLE COMPARATIF 2:

Effet du ritonavir sur la présentation des peptides antigéniques par le CMH de classe 1 à des lymphocytes T cytotoxiques de souris

### 1. Effet du ritonavir sur la lyse médiée par les lymphocytes T cytotoxiques des cellules cibles infectées par LCMV-WE et traitées in vitro avec du ritonavir.

### Méthode :

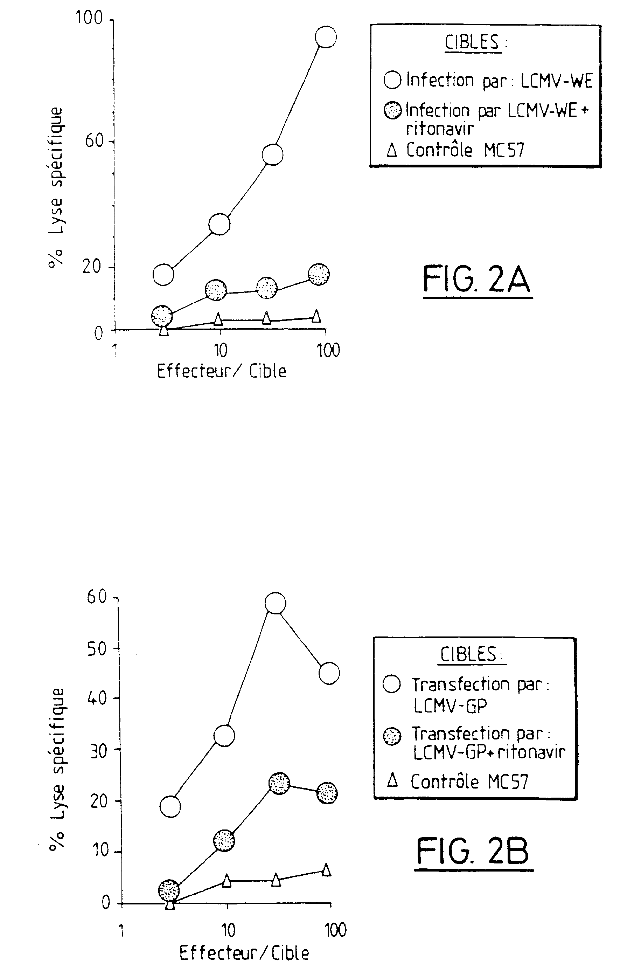

Des lymphocytes T cytotoxiques ont été prepares a partir de spiénocytes de souris infectées par LCMV et restimulés *in vitro* par des cellules sensibilisées, soit avec ie peptide GP33-41 (figure 2A) soit avec le peptide NP396-404 (figure 2B).

Leur activité cytolytique a été testée vis à vis de fibroblastes MC57 (H-2°) infectés par LCMV à une multiplicité d'infection de 0.04 et incubés ou non en présence de ritonavir à 5 µg/ml pendant 36 heures.

L'effet direct du ritonavir sur les lymphocytes T cytotoxiques *in vitro* a été exclu en lavant de manière extensive les cellules cibles avant le test de lyse.

### Résultats :

Ces études *in vitro* sur des cellules présentatrices d'antigènes viraux après infection par un virus indiquent que l'inhibition des réponses des lymphocytes T cytotoxiques intervient au niveau de la présentation de l'antigène. L'incubation des cellules MC57 infectées par ie virus LCMV avec du ritonavir inhibe fortement la lyse par les lymphocytes T cytotoxiques anti-LCMV (figure 2A). Un effet inhibiteur similaire a également été observe sur des cellules MC 57 traitées au ntonavir et exprimant par transfection la glycoprotéine du LCMV (figure 2B).

### EXEMPLE COMPARATIF 3 :

### Effet du ritonavir sur la présentation des peptides antigéniques par le CMH I à des clones de lymphocytes T humains

### Méthode :

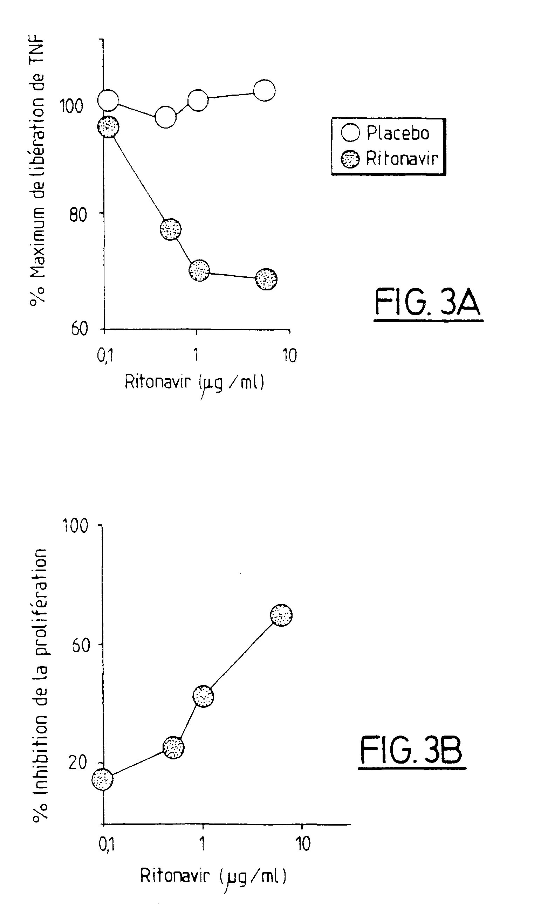

a) Dans cette expérience, on a utilisé des cellules M113 et M77-84 qui sont respectivement une lignée de mélanome HLA-A2 présentant l'antigène MART-1 et un clone de lymphocytes T cytotoxiques CD8+ reconnaissant cet antigène (Y. Kawakami et al, J. Exp. Med., 180. 347 (1994) : N. Gervois. Y. Guilloux, E. Diez, and F. Jotereau. J. Exp. Med.. 183. 2403-7 (1996)). Les cellules M113 ont été cultivées pendant vingt quatre heures avec différentes concentrations de ritonavir puis resuspendues et fixées dans du paraformaldehyde. Comme contrôle, les mêmes concentrations en indinavir ont été utilisées. La production de TNFα par les lymphocytes T M77-84 répondeurs dans le surnageant a été mesurée et est exprimée sur la figure 3 en pourcentage de libération maximale obtenue avec des cellules M113 non traitées.
b) Les cellules M113 ont été cultivées pendant 24 heures en présence de ritonavir (0.1-5 µg/ml) et ensuite utilisées comme stimulatrices dans un test de prolifération avec les lymphocytes T M77-84 comme cellules répondeuses. La prolifération a été mesurée après 48 heures en présence de thymidine ajoutée à 1 µCi/puits pendant les dernières dix-huit heures (figure 3B).

### Résultats :

Ces expériences utilisant des clones de lymphocytes T cytotoxiques humains diriges contre des antigènes de mélanomes ont conduit à des résultats similaires des expériences précédentes exposées ci-dessus. Les figures 3A et 3B montrent l'inhibition de la présentation de l'antigène MART-1 de mélanomes humains par des cellules croissant en présence de ritonavir a des lymphocytes T CD8⁺. L'inhibition est dose-dépendante et est efficace à des doses thérapeutiques, comme cela a été observé lorsque les cellules présentatrices d'antigènes sont cultivées dans du sérum obtenu à partir de volontaires VIH négatifs après ingestion d'une dose orale unique de 500 mg de ritonavir. On a observé jusqu'à 70 % d'inhibition de ia prolifération avec des sérums prélevés 3 heures après l'ingestion, correspondant au pic de concentration en ritonavir plasmatique.

### EXEMPLE COMPARATIF 4 :

### Effet du ritonavir sur l'activité "chymotrypsin-like" du protéasome

### 1. Effet du ritonavir sur la dégradation des protéines à courte durée de vie.

### Méthode :

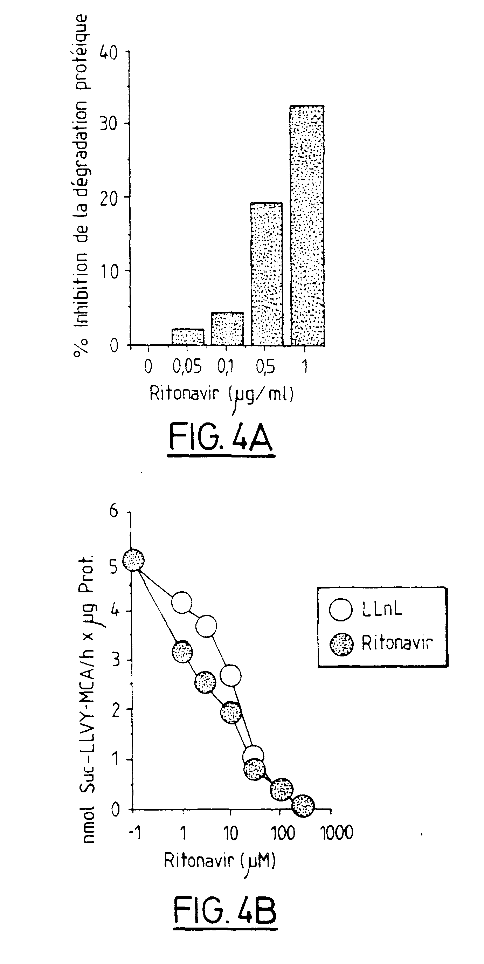

Pour ces analyses, les cellules ont été cultivées une nuit en présence d'une concentration variable de ritonavir, préincubées dans un milieu sans méthionine ni cystéine pendant 1 heure, soumis a un marquage avec de la ³⁵S méthionine et de la ³⁵S cystéine pendant une heure, lavees dans du PBS et soumises à une chasse pendant une heure. Après une heure, du TCA (acide trichlorcacétique à 10 % final) a été ajouté dans le surnageant et la radioactivité du surnageant non précipité par le TCA a été quantifiée avec un Lumaplate (Topcount Packard) (figure 4A).

### Résultats

Le ritonavir inhibe de manière dose-dépendante la dégradation de protéines à courte durée de vie, procédé connu pour être principalement dépendant de la voie de l'ubiquitine protéasome. Ces résultats suggèrent fortement que l'activité du protéasome est modifiée par le ritonavir.

### 2. Effet du ritonavir sur la protéasome.

### Méthode :

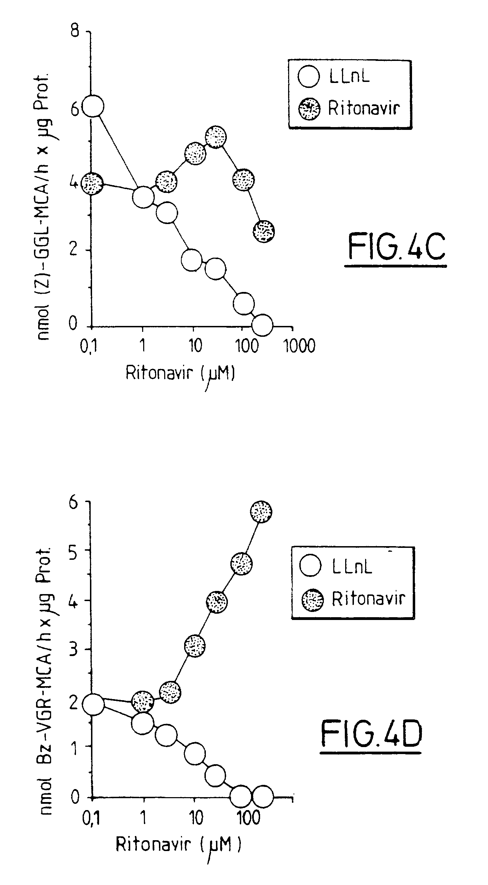

Les figures 4B, 4C et 4D montrent l'hydrolyse de substrat fluorogene (100 µM Suc-LLVY-MCA. 100 uM (Z)-GGL-MCA et 400 µM Bz-VGR-MCA) des protéasomes 20S isolés à partir de fibroblastes B8 murins, en fonction de concentrations variables en ritonavir et en LLnL (N-acétyl-L-leucinyl-L-leucinal-L-norleucinal, inhibiteur connu du protéasome). Les résultats sont rapportés pour une heure de digestion avec du protéasome 20S (500 ng) dans un volume final de 100 µl. Les valeurs ont été localisées dans un domaine linéaire de détection et sont les moyennes de triplicatas avec des erreurs standards inférieures à 3 %.

### Résultats :

Les résultats présentés sur les figures 4B. 4C et 4D montrent que le ritonavir est un inhibiteur puissant de l'hydrolyse médiée par le protéasome du substrat fluorogène Suc-LLVY-MCA (coupure du côté carboxy de la tyrosine). En revanche, l'hydrolyse du substrat (Z)-GGL-MCA (coupure du côté carboxy la leucine) a été à peine affectée, et le clivage du substrat Bz-VGR-MCA (coupure du côté carboxy de l'arginine, par une activité du type « trypsine ») a été augmenté de manière conséquente. Cette inhibition sélective par !e ritonavir contraste avec l'inhibition par l'aldéhyde de peptide LLnL du protéasome, connu pour bloquer de manière covalente tous les sites actifs des protéasomes 20S, et qui inhibe de manière similaire la protéolyse des trois substrats fluorogènes testés (V. Cerundolo et al, Eur. J. Immunol. 27. 336 (1997) ; A. Vinitsky et al, J. Immunol. 159. 554 (1997) ; M. groll et al. Nature, 386, 463 (1997).

La maturation des molécules de classe I dans le réticulum endoplasmique pour acquérir une résistance à l'endoglycosidase-H et l'expression à la surface des molécules de classe I n'ont pas été bloquées de façon significative par un traitement au ritonavir, ce qui est en accord avec une inhibition sélective.

### 3. Comparaison de quatre inhibiteurs de protéase VIH-1.

### Méthode :

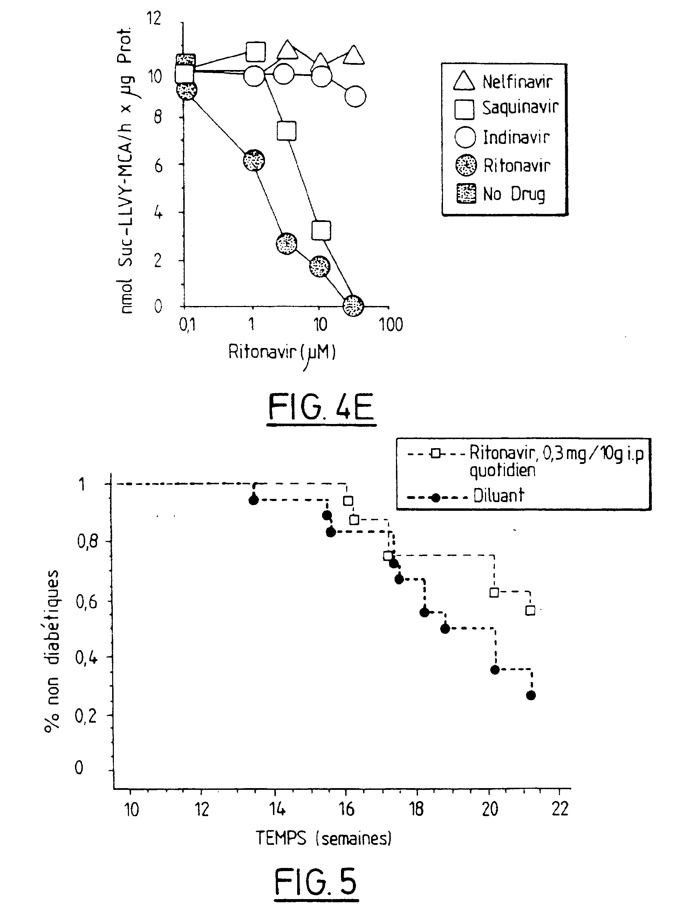

L'hydrolyse de Suc-LLVY-MCA par les protéasomes murins 20S a été testée selon la méthode décrite dans l'exemple 4.2. dans des conditions identiques à l'exception du fait qu'un lot différent de protéasomes purifiés a été utilisé. L'hydrolyse de Suc-LLVY-MCA a été mesurée en présence de concentrations croissantes en ritonavir, nelfinavir (Roche), saquinavir (mesylate) et indinavir (principe actif du Crixivan® commercialisé par Merck et Sharo and Dohmei. Les inhibiteurs de protéase VIH ont préalablement été dissous dans l'éthanol, le méthanol. le DMSO et l'eau respectivement, pour une utilisation *in vitro* (figure 4E).

### Résultats :

A part le ritonavir, le mésylate de saquinavir inhibe l'activité "chimotrypsin-like", tandis qu'aucune inhibition n est observée avec l'indinavir et le nelfinavir. Le gonflement du coussinet après une injection de LCMV et la lyse directe *ex vivo* après infection systémique n'ont pas été inhibées par l'indinavir ou le nelfinavir. En revanche, une inhibition du gonflement des coussinets plantaires peut être observée avec le saquinavir (jusqu'à 73 % d'inhibition au 7^{ème} jour après le traitement avec une dose orale de 4 mg par jour de saquinavir).

En conclusion, l'inhibition spécifique et sélective du proteasome par le ritonavir ainsi que par le saquinavir expliquerait les effets modulateurs de la présentation d antigène observée *in vivo*.

### Conclusion :

Les souris infectées par injection du LCMV dans le coussinet plantaire développent une inflammation locale avec oedème réactionnel qui est en relation directe avec l'intensité de la réponse des lymphocytes T cytotoxiques. Les souris ne souffrent plus de réaction inflammatoire au niveau de l'injection lorsqu'elles reçoivent du ritonavir par voie intrapéritonéale ou orale et le nombre de lymphocytes T CD8 spléniques n'est pas augmenté chez les souris traitées. Les splénocytes de ces souris traitées ont une activité cytolytique très faible vis a vis de cellules cibles sensibilisées par les peptides du LCMV. Simultanément, la clairance des virus est retardée et diminuée.

Les études *in vitro* avec des cellules infectées ou transfectées et tumorales indiquent que la modulation de la réponse cvtotoxique s'exerce au niveau de la présentation des antigènes. L'action de ces inhibiteurs est observée aussi bien avec des cellules humaines ou murines et à des concentrations obtenues avec les doses thérapeutiques données au cours des infections par le virus de l'immunodéficience humaine.

*In vitro,* la dégradation des protéines de courte durée de vie, un phénomène principalement assuré par le protéasome, est diminuée en présence de ritonavir. Les préparations purifiées de protéasome modifient leur activité enzymatique en présence de ritonavir. La genèse des peptides endogenes capables de se fixer sur le complexe majeur d'histocompatibilité de classe (CMH-1) est donc modifiée, ce qui entraîne une modification du recrutement et de la stimulation des lymphocytes T CD8. L'action du ritonavir a été observée avec différentes cellules présentatrices d'antigènes humaines ou murines vis à vis de différentes cellules T CD8. Une modification de l'activité enzymatique du protéasome est observée également avec le mésyiate de saquinavir.

### EXEMPLE 5 COMPARATIF:

### Effet du ritonavir dans le traitement du diabète de type 1 Méthode :

Du ritonavir (0.6 mg pour 10 g de poids corporel par jour) ou un placébo (tampon phosphate PBS) a été administré par voie intrapéritonéale à des souris NOD. Les îlots pancréatiques ont été recueillis et cultivés pendant 24 heures en présence ou en l'absence de 2,5 µg/ml de ritonavir. Les cellules ont ensuite été co-cultivées avec des hybridomes restreints par le CMH-1 (FT6.9 et FT7.9 pendant 24 heures). La quantité d'IL-2 a été dosée dans le surnageant.

### Résultats :

Les tableaux suivants présentent les quantités d'IL-2 mesurées par un test de prolifération (cpm x 10⁻³).
a) Hybridome ETG.9 (CMH-I) 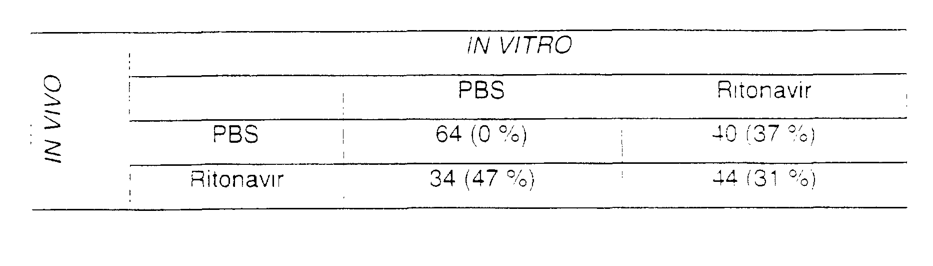
b) Hybridome FT7.9 (CMH-I)

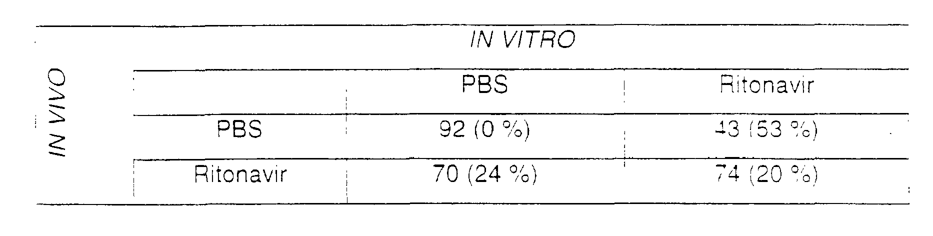

Dans ce modèle de diabète murin NOD, les cellules de Langerhans isolées de souris traitées par le ritonavir ne sont plus reconnues *ex vivo* par des clones T cytotoxiques.

En outre, comme le montre la figure 5, le traitement par le ritonavir retarde l'apparition du diabète de type 1 chez les souris NOD.

### EXEMPLE 6 COMPARATIF :

### Effet du ritonavir dans le traitement de l'encéphalite allergique expérimentale du rat et de la souris, modèle de la sclérose en plaques humaine

### a) chez le rat :

### Méthode :

L encéphalite allergique expérimentale chez le rat est le seul modèle de sclérose en plaques chez l'homme. On administre de la MBP de cochon d'Inde (Myelin Basic Protein ou protéine de liaison à la myéline) à des rats femelles Lewis de 7 semaines, par une injection sous-cutanée dans le coussinet plantaire de 50 µg de MBP dans 100 µl d'adjuvant complet de Freund supplémenté de 4 µg/ml de mycobactéries. On administre en outre à ces rats. quotidiennement, soit du ritonavir 1 mg, 5 mg ou 10 mg, par animal, soit un placebo (tampon phosphate) de l'injection de MBP jusqu'à la guérison des animaux non traités par le ritonavir. Les scores cliniques mesurant la progression de l'encéphalite allergique expérimentale sont les suivants :
1 : faiblesse des mouvements de la queue :
2 : paralysie incomplète du train arrière ;
3 : paralysie complète du train arrière :
4 : paralysie incomplète du train antérieur ; et
5 : immobilité totale.

Environ huit jours après cette immunisation avec la MBP. les rats contrôle commencent à montrer les premières étapes de paralysie (transitoire) due à la réponse immunitaire provoquée par l'administration de myéline.

### Résultats :

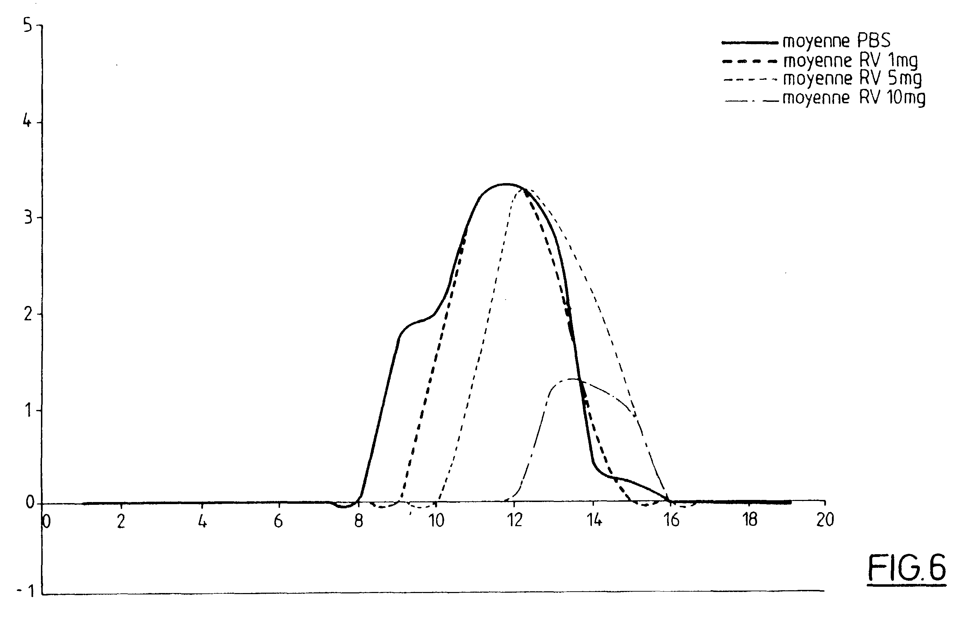

L'apparition des premiers signes de paralysie est retardée chez les rats traités par du ritonavir (1 et 5 mg). Parmi les rats traités avec 10 mg de ritonavir, deux n'ont pas développé la maladie, et les autres rats n'ont présenté que les symptômes atténués de l'EAE (score clinique : 1) (figure 6).

### b) chez la souris :

Des résultats similaires à ceux décrit chez le rat ont été obtenus chez la souris. Des informations supplémentaires ont été enregistrées qui montrent la possibilite d'un transfert de la protection induite par le retonavir.

### Méthodes et résultats :

Dans cette expérience, des souris receveuses saines non traitées au ritonavir reçoivent par voie intraveineuse et/ou péritoneale des cellules des ganglions lymphatiques et de la rate provenant de souris donneuses traitées au ritonavir et avant subi le protocole d'induction d'EAE précédemment décrit dans l'exemple a) et adapté à la souris.

Les ganglions poplités, inguinaux et mésentériques et la rate sont prélevés à J10 après l'injection de MBP, mis en culture en présence de 50 µg/ml de MBP. Au troisième jour de culture, les cellules non adhérentes sont récupérées, passées sur Ficoll et injectées aux souris receveuses (10 x 10⁶ cellules injectées). Le protocole d'induction d'EAE à J30 après ie transfert des cellules ne provoque aucun symptôme d'EAE chez ces animaux receveurs.

### Conclusion :

Le traitement par le ritonavir protège les souris et les rats de l'EAE. Cette protection peut être transférée par l'intermédiaire des cellules des ganglions lymphatiques et de la rate des souris traitées à des souris receveuses non traitées au ritonavir.

### EXEMPLE 7 :

### Effet du ritonavir dans le traitement de l'hépatite C

### 1. Objectif

Le virus de l'hépatite C (VHC) est le principal agent étiologique responsable des hépatites non-A non-B post-transfusionnelles et sporadiques. Au moins 70 % des infections deviennent chroniques évoluant très fréquemment vers la cirrhose et le cancer. L'infection par le VHC est un problème majeur de santé publique et les traitements à base d'interféron, qui sont les plus largement utilisées, ne sont efficaces que dans 25 % des cas.

Les lymphocytes T CD8 anti-VHC prédominent dans les infiltrats inflammatoires hépatiques au cours des infections chroniques. Leur activation accrue provoque à la fois une importante destruction des hépatocytes conduisant à l'insuffisance hépatique et une immunosuppression qui empêche la clairance totale du virus. Un contrôle spécifique de l'activation des lymphocytes T CD8 cytotoxiques permettrait d'atténuer l'immunopathologie de l'infection VHC en levant l'immunosuppression et réduisant la destruction hépatique.

Les mécanismes responsables des dégâts hépatiques lors de l'infection par le virus de l'hépatite C (VHC) sont mal connus. L'observation clinique que des courts traitements avec des corticostéroïdes réduisent les taux sériques d'aminotransférases malgré une aggravation de la virémie, suggère que la réponse immunitaire joue un rôle important dans la destruction des hépatocytes. Le fait que la majorité des patients infectés par le VHC progresse vers la chronicité indique que la présence de lymphocytes T cytotoxiques anti-VHC ne suffit pas à éliminer le virus. Bien que les lymphocytes T CD8 soient présents en grand nombre dans les infiltrats inflammatoires, leur rôle à la fois dans la protection contre le virus et dans la destruction des tissus hépatiques reste confus. Les patients avec une forte activité des lymphocytes T cytotoxiques anti-VHC ont certes une virémie plus basse mais iis ont aussi des taux plus élevés de transaminases et développent une maladie plus active (Nelson D.R. et al, J. of Immunol. 158, 1473 (1997). A l'inverse, les patients avec une forte réponse CD4 anti-VHC présentent moins fréquemment des signes cliniques et histologiques de maladies hépatiques. Par conséquent, la balance entre le clairance virale et la destruction hépatique est fortement liée à l'activité relative des populations intrahépatiques de lymphocytes T CD4 et CD8 spécifiques du VHC.

Chez un patient infecté chroniquement par le VHC, la déplétion des lymphocytes T CD8 par des anticorps monoclonaux anti-CD8 a des effets bénéfiques (Kiefersauer S. et al, J. of Immunol., 159. 4046 (1997). Après ce traitement, les transaminases ont fortement diminué, l'inflammation hépatique est réduite et la réponse proliférative aux antigènes du VHC est restaurée. Tsai et al ont également montré que la déplétion *in vitro* des lymphocytes T CD8 à partir du sang périphérique autorisait une réponse proliférative aux antigènes du VHC (Tsai et al, J. Hepatol. 21. 403 (1993).

La réponse proliférative est un élément déterminant de la clairance virale lors de la phase d'infection aiguë et une forte réponse T CD4 est associée à l'élimination du virus. Cette réponse est faible ou absente chez les patients qui progressent vers la chronicité ou qui manifestent des signes cliniques de chronicité (Diepolder H et al, Lancet. 346, 1006 (1995).

Le modèle murin d'infection chronique par le LCMV ressemble, par certains aspects, à l'infection chronique par le VHC. Dans ce modèle, les lymphocytes T CD8 peuvent non seulement nuire à l'élimination du LCMV mais en plus ils rendent les animaux incapables de lutter contre une nouvelle infection par le VSV (Dunlop M et Blanden R, J. exp. Med. 145, 1131 (1977) ; Odermatt B et al, Natl Acad. Sci. USA 88, 8252 (1991)). La déplétion des lymphocytes T CD8 par des anticorps monoclonaux anti-CD8 s'accompagne d'une restauration complète de la réponse immunitaire contre le VSV (Leist T.P., E. Ruedi et R.M. Zinkernagel, J. Exp. Med. 167, 1749 (1988)). De plus, les lymphocytes T cytotoxiques peuvent détruire les cellules infectées telles que les cellules présentatrices d'antigène, les lymphocytes T et ies lymphocytes B producteurs d'anticorps neutralisants qui sont essentielles pour développer une réponse protectrice (Odermatt B et al, Natl Acad. Sci. USA 88. 8252 (1991) : Leist T.P.. E. Ruedi et R.M. Zinkernagel, J. Exp. Med. 167, 1749 (1988)).

La modulation de l'activité CD8 anti-VHC pourrait donc être une bonne stratégie pour favoriser le développement d'une immunité humorale protectrice.

### 2. Cas clinique

Les profondes modifications de la réponse cytotoxique T CD8 ouvrent une nouvelle voie thérapeutique pour les infections chroniques avec des virus non-cytopathogènes. En effet, l'exemple du LCMV montre que l'utilisation du ritonavir dans l'infection chronique par le virus de l'hépatite C devrait entraîner une diminution de la réponse cytotoxique et des effets immuno-pathologiques avec des conséquences cliniques favorables.

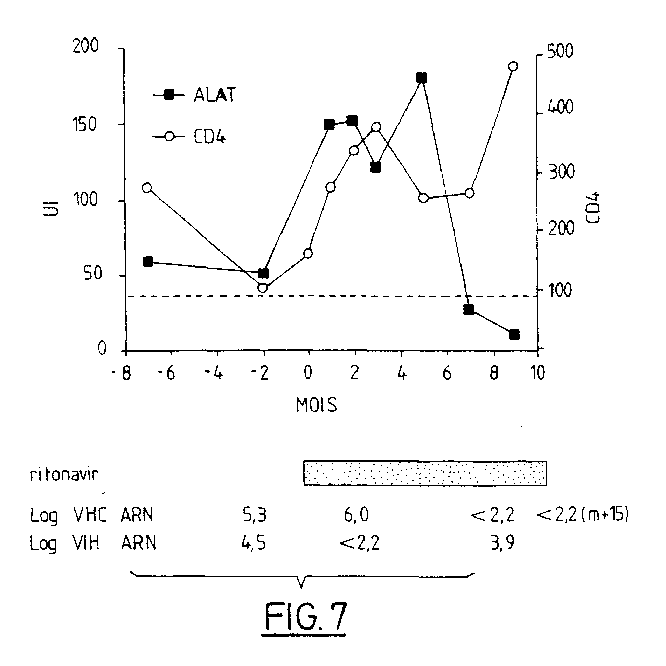

Un patient toxicomane est suivi pour une double infection par le VIH et le VHC, découverte en 1986 mais sans doute antérieure à cette date. L'évolution de l'infection par le VIH est lente avec une virémie établie autour de 4 Log HIV ARN copies/ml. Le taux de lymphocytes T CD4 a chuté avec une première infection opportuniste pulmonaire. La virémie VHC oscillait autour de 5 à 6 Log HCV ARN copies/ml et le taux sérique de transaminases était modérément élevé (2 ou 3 fois la valeur normale). Un inhibiteur de protéases du VIH, le ritonavir, a été introduit dans la thérapeutique du patient et poursuivi depuis lors. La virémie VIH est devenue indécelable pendant les deux mois qui ont suivi le début du traitement avant de revenir au niveau antérieur dès le quatrième mois. Le taux de lymphocytes T CD4 a augmenté pendant cette période et a continué à augmenter même après la réapparition de la virémie VIH. En revanche, la virémie VHC est devenue négative au 7^{ème} mois après l'introduction du ritonavir (figure 7), et l'est toujours deux ans après.

### 3. Etude rétrospective de l'évolution de l'infection par le VHC chez des patients sidéens traités au ritonavir ou à l'indinavir.

Chez des patients infectés par le VIH-1, l'effet du ritonavir sur la réponse immunitaire peut résulter de son activité antirétroviraie et de la réduction de l'activité des lymphocytes T cytotoxiques (CTL) et de l'immunopathologie. Une étude rétrospective de patients coinfectés par les virus VHC et VIH a été menée pour comparer la charge virale VHC. la fonction hépatique et le titre anticorps anti-VHC chez des patients recevant du ritonavir et du saquinavir ou de l'indinavir durant les quatre premiers mois de la thérapie. Les patients enrôlés (41) avaient une infection VHC chronique prouvée par la présence d'anticorps anti-VHC et d'ARN sérique VHC détectés par RT-PCR. Dix-huit patients ont reçu du ritonavir seul (soit 600 mg deux fois par jour), trois ont reçu du ritonavir et du saquinavir (800 mg par jour), 20 ont reçu de l'indinavir (800 mg trois fois par jour). La population contrôle était constituée de 105 patients sans anticorps anti-VHC ou anti-VHB détectables et sans infection opportuniste, mais recevant du ritonavir pour la première fois pendant au moins quatre mois.

Initialement, les patients co-infectés montraient les symptômes cliniques, une charge virale VIH, une numération en lymphocytes T et des valeurs biologiques similaires. Une cytolyse hépatique modérée existait chez les deux groupes (87 +/- 61 IU/l pour l'indinavir et 54 +/- 28 IU/l pour le ritonavir). Les charges virales VHC et la distribution génotypique VHC entre les deux groupes étaient similaires. Chez les patients infectés, les concentrations en ALAT ont augmenté de manière plus fréquente chez ceux prenant du ritonavir (57 % : 12/21 patients) que chez ceux prenant de l'indinavir (10 % : 2/20 patients ; p < 0.002). Dans le groupe ritonavir, on a pu observer un pic de concentration ALAT durant le troisième mois avec une valeur maximale moyenne de 418 IU/I (écart : 96-1570).

Dans la population contrôle, une augmentation des .concentrations en ALAT n'a été observée que chez 12 patients sur 105 (11.4 %, p=10⁻⁶) indiquant que la très haute fréquence de patients infectés par le VHC et souffrant d'une hépatite cytolytique durant le traitement par le ritonavir n'est pas liée à la toxicité du médicament mais résulte de son action sur les cellules infectées par le VHC soit directement soit par la modulation de la réponse immunaire.

Le tableau 1 ci-après présente une comparaison des marqueurs biologiques des patients infectés par le VHC avec ou sans augmentation des transaminases durant le traitement par le ritonavir (groupes I et II respectivement) et chez les patients traités par de l'indinavir sans élévation d'ALAT (groupe III).

Des calculs statistiques avec le groupe IV n'ont pas été réalisés en raison du petit nombre de patients. II s'agissait de deux patients avec une hépatite cytolytique associée avec une importante augmentation de la charge virale VHC (+ 3.57 log₁₀ de copies d'ARN/ml chez un patient et + 0.5 log₁₀ de copies d'ARN/ml chez le second patient).

Les variations des concentrations d'ARN VHC et des index d'anticorps ont été mesurées sur des paires de sérum conservées (10 paires dans le groupe I. 6 dans le groupe II, 10 dans le groupe III et 2 dans le groupe IV). Les sérums ont été recueillis initialement et aux semaines 6.8 et 7.2 pour les groupes I et III respectivement (p >0.2). Les concentrations en ARN VIH et VHC sont présentées sous la forme de log₁₀ de copies d'ARN/ml et les numérations en lymphocytes T sous la forme de cellules/mm³, Les concentrations en ALAT sont exprimées en unités internationales/l.

Les anticorps anti-VHC ont été quantifiés par la version 3.0 du système Axsym VHC (Abott) et les variations des titres d'anticorps anti-VHC des sérums de patients (Ac index) sont exprimées par le rapport des densités optiques.

Les variations de la charge virale VIH et de la numération des lymphocytes T ont été similaires chez les trois groupes I, II et III. La charge virale VHC a augmenté chez tous les groupes avec + 0.61 log₁₀ de copies d'ARN/ml pour le groupe I comparé à + 0,41 log₁₀ de copies d'ARN/ml dans le groupe III (p < 0.1) et + 0.22 log₁₀ de copies/ml dans le groupe II (p = 0.065). Une augmentation de la production d'anticorps anti-VHC n'a été observée que chez le groupe I (p < 0.05 entre les groupes I et III),

Ainsi, le titre d'anticorps n'augmente que chez les patients présentant une cytolyse lors de la thérapie par ritonavir. Aucune augmentation des titres d'anticorps n'a été détectée sous indinavir même lorsqu'une forte augmentation de la virémie VHC a été observée, indiquant qu'une stimulation antigénique abondante n'est pas suffisante pour stimuler la réponse humorale anti-VHC. L'amélioration de la réponse humorale précoce pendant un traitement par le ritonavir peut être la conséquence de la modulation de l'activité CTL puisque, lors des infections par HBV et LCMV, Il a été montré que les lymphocytes T cytotoxiques anti-viraux lysaient les lymphocytes B producteurs d'anticorps neutralisants (Planz et al, 1996, Nature, 382:726-729 ; Barnaba et al, 1990, Nature. 345:258-260).

La cytolyse hépatique transitoire observée quelques jours après le début du traitement par ritonavir chez les patients atteints d'hépatite C pourrait ainsi résulter d'une facilitation de l'apoptose des hépatocytes infectés par le VHC. Cette observation ouvre la voie à l'application de ces molécules (ritonavir et saquinavir) à des fins de modulation de l'apoptose.

**Tableau 1 : Variations des valeurs biologiques chez des groupes de patients infectés par le VHC classées selon l'inhibiteur de protéase du VIH-1 utilisé et la cytolyse hépatique.**

| | Groupe I RTN, cytolyse + | Groupe II RTN, cytolyse - | P I *versus* II | Groupe III IDN. cytolyse - | P I *versus* III | Groupe IV IDN, cytolyse - |
|---|---|---|---|---|---|---|
| | N=12 | N=9 | | N=18 | | N=2 |
| CD4 | 109.4(+/-113) | 88.3(+/-170) | p = 0.36 | 21.1 (+/-68) | p = 0,085 | 29,5 |
| CD8 | 203,6 (+/-650) | 281,8 (+/-394) | p = 0,95 | 9.2 (+/-222) | p = 0,22 | 203.5 |
| HCV-VL | 0,61 (+/-0.3) | 0.22 (+/-0.35) | p = 0,065 | 0.41 (+/-0.79 | p < 0.01 | 2.25 |
| Ac index | 2,19 (+/-1.68) | 0,98 (+/-0,24) | p = 0,085 | 1,01 (+/-0.13) | p < 0,05 | 0,98 |

| | | | | | | |
|---|---|---|---|---|---|---|
| N= nombre d'individus par groupe. | | | | | | |
| RTN et IDN signifient ritonavir et indinavir respectivement. | | | | | | |

## Revendications

1. Utilisation d'au moins un composé inhibiteur de la protéase du virus de l'immunodéficience humaine (VIH) choisi parmi le ritonavir, le saquinavir, ou de leurs sels pharmaceutiquement acceptables, en association avec un véhicule pharmaceutiquement acceptable, pour la fabrication d'un médicament pour la prévention et/ou le traitement d'une infection par le virus de l'hépatite C.

2. Utilisation selon la revendication 1, dans laquelle ledit médicament est sous la forme d'une composition pour administration orale.

3. Utilisation selon la revendication 1, dans laquelle ledit médicament est sous la forme d'une composition pour administration parentérale.

4. Utilisation selon l'une des revendications 1 à 3, dans laquelle ledit médicament contient de 1 à 2 000 mg dudit composé inhibiteur de la protéase du VIH.

5. Utilisation selon la revendication 4, dans laquelle ledit médicament contient de 100 à 1500 mg dudit composé inhibiteur de la protéase du VIH.

## Patentansprüche

1. Verwendung mindestens einer Verbindung, welche die Protease des humanen Immunschwächevirus (HIV) inhibiert und aus Ritonavir, Saquinavir oder deren pharmazeutisch verträglichen Salzen ausgewählt ist, zusammen mit einem pharmazeutisch verträglichen Träger zur Herstellung eines Arzneimittels für die Verhütung und/oder Behandlung einer Infektion mit dem Hepatitis-C-Virus.

2. Verwendung nach Anspruch 1, bei welcher das Arzneimittel in Form einer Zusammensetzung für die orale Verabreichung vorliegt.

3. Verwendung nach Anspruch 1, bei welcher das Arzneimittel in Form einer Zusammensetzung für die parenterale Verabreichung vorliegt.

4. Verwendung nach einem der Ansprüche 1 bis 3, bei welcher das Arzneimittel 1 bis 2 000 mg der die HIV-Protease inhibierenden Verbindung enthält.

5. Verwendung nach Anspruch 4, bei welcher das Arzneimittel 100 bis 1 500 mg der die HIV-Protease inhibierenden Verbindung enthält.

## Claims

1. Use of at least one human immunodeficiency virus (HIV) protease inhibitor selected from ritonavir, saquinavir, or pharmaceutically acceptable salts thereof, in association with a pharmaceutically acceptable vehicle, for the production of a medication for the prevention and/or treatment of an infection through the hepatitis C virus.

2. Use according to Claim 1, wherein said medication is in the form of a composition for oral administration.

3. Use according to Claim 1, wherein said medication is in the form of a composition for parenteral administration.

4. Use according to one of Claims 1 to 3, wherein said medication contains 1 to 2000 mg of said HIV protease inhibitor.

5. Use according to Claim 4, wherein said medication contains 100 to 1500 mg of said HIV protease inhibitor.
